# EUROPÄISCHE PATENTSCHRIFT

(11) **EP 1 396 747 B1**
(45) Veröffentlichungstag und Bekanntmachung des Hinweises auf die Patenterteilung: **26.07.2006**
(21) Anmeldenummer: 03019329.6
(22) Anmeldetag: 27.08.2003
(51) Int. Cl.: G02B 21/00, A61B 3/13

(54) **Beleuchtung für Operationsmikroskope zum Schutz von menschlichem Gewebe**
Illumination for a surgical microscope in order to protect human tissue
Dispositif d'éclairage dans un microscope chirurgical pour la protection du tissu humain

(30) Priorität: 06.09.2002 DE 10241261
(43) Veröffentlichungstag der Anmeldung: 10.03.2004
(73) Patentinhaber: Leica Microsystems (Schweiz) AG, 9435 Heerbrugg (CH)
(72) Erfinder: Sander, Ulrich, Dr., 9445 Rebstein (CH)
(74) Vertreter: Rosenich, Paul

(56) Entgegenhaltungen:
- EP-A- 0 482 340
- EP-A- 1 231 496
- US-A- 4 834 528
- US-A- 5 841 149
- US-B1- 6 350 031

## Beschreibung

Operationsmikroskope senden zur Beleuchtung des Operationsfeldes sehr helles Licht aus. Dieses kann bei Augenoperationen zu Schädigungen der Netzhaut oder der Kornea des Patientenauges führen. Es wurden intensive Anstrengungen unternommen, dieses zu verhindern.

### Folgende Möglichkeiten kennt man aus dem Stand der Technik:

US-A-4715704 offenbart unter dem Namen einer sogenannten "Lichtfalle" eine Retinaschutzblende. Eine lichtundurchlässige Blende im Beleuchtungsstrahlengang wird in das Objektfeld abgebildet und verdunkelt dort den zentralen Teil des Leuchtfeldes. Der Operateur muss das Patientenauge so ausrichten, dass es in diesem verdunkelten Bereich zu liegen kommt. Das ist vom Operationsablauf her unpraktisch und die Sicht des Operateurs wird eingeschränkt.

Aus DE-A1-19538382 ist eine Schutzeinrichtung bekannt, die über eine Abstandsmessung die Helligkeit reduziert, die Beleuchtung abschaltet oder einen Warnton oder ein -signal abgibt, sobald der Abstand bei eingeschalteter Beleuchtung zum Patientenauge zu gering wird.
Somit wird aber der Schutz des Patientenauges mit einer Reduktion der Objektfeldhelligkeit erkauft.

In DE-A-10108254 wurde eine weitere Schutzvorrichtung vorgestellt, welche mit Hilfe von spektralselektiv wirkenden Filtern die Lichtintensität reduziert. Diese Filter verändern allerdings den Farbeindruck des Beleuchtungslichts, was u. U. unerwünscht sein kann.

Des Weiteren ist in der US-A-4948247 die Kombination eines Mikroskops mit einem Stroboskop vorgesehen, jedoch um von bewegten Objekten ein still stehendes Bild zu erzeugen und außerdem nicht zum Schutz von Patientenaugen ausgelegt ist.

Aus der Druckschrift EP-A-0 482 340 ist ein Operationsmikroskop mit einem variablen Shutter bekannt.

Eine bisher noch nicht optimal gelöste Aufgabe ist es somit: Einerseits muss das Patientenfeld sehr hell ausgeleuchtet werden, andererseits soll so wenig Licht wie möglich auf das Patientenauge fallen.

Aufgabe der vorliegenden Erfindung war es somit, im Rahmen dieses Widerspruchs einen möglichst effizienten Schutz bei bestmöglicher Ausleuchtung zu finden.

Gelöst wurde die Aufgabe durch ein Ophtalmo-Operationsmikroskop mit den Merkmalen nach Anspruch 1 bzw Anspruch 2.

Der Erfinder erkannte, dass diesem Widerspruch mit folgendem erfindungsgemäßen Gedanken Abhilfe geschaffen werden kann:

Es ist nicht nötig, dass eine zeitlich kontinuierlich strahlende Lichtquelle verwendet wird, eine bestimmte gepulste Lichtstrahlung genügt für die gleiche Wahrnehmung der Beleuchtung. Gepulste Lichtstrahlung bringt jedoch weniger Energieeintrag in das Auge. Gemäß dem Talbot-Plateauschen Satz rufen nämlich rasch aufeinanderfolgende periodische Lichtreize oberhalb der Flimmergrenze die gleiche Empfindung wie ein Dauerreiz hervor, bei welchem die in den periodischen Reizen vorhandene Lichtmenge gleichmäßig über die ganze Zeit verteilt ist (H. Schober, "Das Sehen", Bd. II, Leipzig 1958).

Demnach ist für kurz dauernde Lichtreize das Produkt aus Netzhautbeleuchtungsstärke und Reizdauer im Beobachterauge des Operateurs und dementsprechend das Produkt aus Lichtstrom und Reizdauer, also die Lichtmenge, für die Empfindung des Operateurs maßgebend. Dabei ist es gemäß H. Schober gleichgültig, ob die Hellzeiten und die Dunkelzeiten untereinander gleich oder verschieden lang sind; die einzige Bedingung ist die Überschreitung der Flimmergrenze.

Mit zunehmender relativer Länge der Dunkelzeit wird lediglich der Lichteindruck entsprechend der abnehmenden Reizdauer innerhalb der Gesamtzeit schwächer, befolgt aber weiter den Talbot-Plateauschen Satz.

Erfindungsgemäß macht man sich nun diese an sich bekannte Erkenntnis zu Nutze, indem insgesamt weniger Lichtenergie das Patientenauge trifft und es somit geschont wird, der Beobachter jedoch genügend Reizinformation für sein Auge erhält. Die vorliegende Erfindung verwertet also einen Nebeneffekt des Talbot-Plateauschen Satzes dahingehend, dass bei noch gleichbleibender oder annähernd gleichbleibender Wahrnehmung durch den Beobachter weniger Lichtenergie zum Ausleuchten des Patientenauges zur Verfügung steht und letzteres dadurch geschont wird.

Ein auf den ersten Blick ähnlicher Erfindungsgedanke ist in US-A-4782386 verwirklicht, wo eine stroboskopische Beleuchtung für ein Endoskop vorgesehen ist, (wohl) zur Minderung der Erhitzung des beleuchteten Gewebes durch eine herkömmliche, heißere Lampe. Die Vorrichtung ist jedoch nur in Kombination mit einer Kamera geoffenbart und nimmt nicht Bezug auf die Bedingungen eines Beobachterauges und noch viel weniger auf die eines Patientenauges, bzw. auf den Prozess, dass ein Beobachterauge ein Patientenauge betrachtet.

Zur Reduktion der Blendwirkung beim Autofahren ist in den Daimler-Chrysler-News vom 05.04.2000 ein System vorgestellt worden, welches ein gepulstes Laserlicht als (Zusatz-)Scheinwerfer mit einer Videokamera zur Beobachtung der Straße verbindet. Der Fahrer erhält dabei zusätzlich zu dem gesehenen Bild ein von der Videokamera eingeblendetes Bild, welches in den Hellzeiten der Laserlichtscheinwerfer aufgenommen worden ist.

Bei der vorliegenden Erfindung handelt es sich jedoch nicht - wie von Daimler-Chrysler gezeigt - um das Ausnutzen der pulsierenden Eigenschaften eines zusätzlichen Infrarot-Laserlichts, sondern um die Reduktion der direkt ins Patientenauge einfallenden Lichtenergie der Beleuchtung selbst, bei gleichzeitiger ausreichender Reizerzeugung im Beobachterauge. Daimler-Chrysler offenbart hingegen nur ein zusätzliches kameragesteuertes System, welches es gestattet, die normale Beleuchtung schwach zu halten.

Gemäß einer Ausgestaltung kann der gepulste Beleuchtungsstrahl auch dadurch erzeugt werden, dass eine herkömmliche Beleuchtung verwendet wird, deren Beleuchtungsstrahl mittels eines Shutterrades gepulst wird.

Der Shutter hierfür kann elektromechanisch ausgestaltet sein oder ein optoelektronischer, z.B. elektrochromer oder LCD-Shutter sein.

Eine Ausgestaltung der Erfindung sendet dadurch weniger Lichtenergie in das Patientenauge, indem das Licht eines Lasers (z.B. Weißlichtlasers) in das Patientenauge gescannt wird. Solange dabei die Wiederholung des Scannens mit einer Frequenz geschieht, die oberhalb der Flimmergrenze des Beobachterauges liegt, nimmt auch bei dieser Technik der Beobachter eine unverminderte Ausleuchtung bei verminderter Energieeinbringung in das Patientenauge wahr. Das Scannen des Laserstrahls kann hierbei sowohl mittels eines optomechanischen Spiegels geschehen, als aber auch mittels eines fixen "2D-reflective displays" (auch "micro mirror" genannt), welches sich der Nanotechnologie bedient, um den Laserstrahl oder einen beliebigen Lichtstrahl wandern zu lassen, indem die einzelnen Spiegelelemente sukzessive in den aktiven Spiegelmodus fallen.

Zusätzlich zu dieser beschriebenen Technologie kann der Laserstrahl auch noch gepulst werden, sei es selbst oder mittels eines Shutters, sodass auch damit eine weitere Möglichkeit zur Verringerung der Energieeinbringung in das Patientenauge geschaffen ist.

Im Falle der Verwendung eines Mikrospiegels, welcher sich kleinster, hintereinander geschalteter Teilspiegel bedient, um den Laserstrahl wandern zu lassen, ist eine herkömmliche Beleuchtungsquelle, die den Mikrospiegel flächig bestrahlt, auch denkbar.

Des Weiteren soll auch im Rahmen der Offenbarung dieser Anmeldung die Kombination eines von vornherein gepulsten Lichts (sei es durch ein Stroboskop oder durch ein zerhacktes/geshuttetes kontinuierliches Licht erzeugt) mit einem optomechanischen Spiegel oder einem Mikrospiegel liegen. Demzufolge würde eine Verringerung der Energieeinbringung in das Patientenauge sowohl durch die Pulsation als auch durch das Scannen bewerkstelligt werden.

Sämtliche hiermit geoffenbarten Schutzbeleuchtungen sollen sowohl für afokale (Strahlengänge gehen durch ein gemeinsames Hauptobjektiv) als auch für Systeme nach Greenough (separate Objektive für jeden stereoskopischen Strahlengang) Anwendung finden.

Weitere Ausbildungen der Erfindung sind in den Figuren und in den Patentansprüchen angegeben.

Die Bezugszeichenliste ist Bestandteil der Offenbarung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche Bauteile an.

Es zeigen dabei
Fig.1a - ein Diagramm einer zeitlich kontinuierlichen Strahlung einer herkömmlichen Lichtquelle,
Fig.1b - ein Diagramm einer gepulsten Strahlung,
Fig.2a - einen schematischen Aufbau eines gegenbeispiels eines Mikroskops mit herkömmlicher Beleuchtung,
Fig.2b - einen schematischen Aufbau eines gegenbeispiels eines Mikroskops mit einer Stroboskoplampe,
Fig.2c - einen schematischen Aufbau eines Ausführungsbeispiels eines erfindungsgemäßen Mikroskops mit einem Laser und einem optomechanischen Spiegel,
Fig.2d - einen schematischen Aufbau eines Ausführungsbeispiels eines erfindungsgemäßen Mikroskops mit einem Laser und einem Mikrospiegel,
Fig.2e - einen schematischen Aufbau eines Ausführungsbeispiels eines erfindungsgemäßen Mikroskops mit einer herkömmlichen Beleuchtung und einem Mikrospiegel und
Fig.2f - einen schematischen Aufbau eines Ausführungsbeispiels eines erfindungsgemäßen Mikroskops mit einer Stroboskoplampe und einem Mikrospiegel.

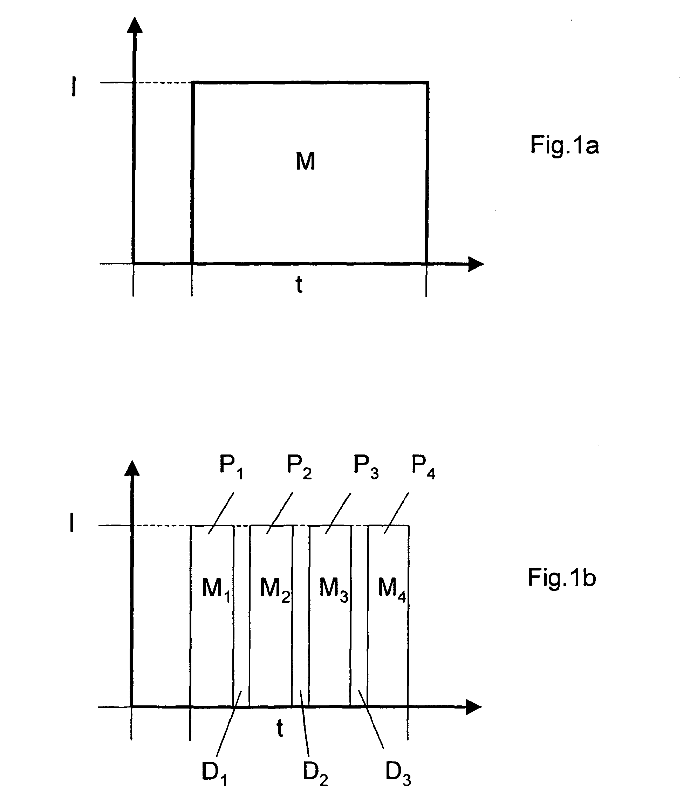

Das Diagramm in Fig.1a stellt die Strahlung einer herkömmlichen Lichtquelle dar, wobei auf der X-Achse der zeitliche Verlauf und auf der Y-Achse die Intensität der Strahlung aufgezeichnet ist. Dabei wird deutlich, dass die Strahlung einer herkömmlichen Lichtquelle über ein gesamtes Zeitintervall t mit einer gleichbleibenden Intensität I erfolgt. Daraus ergibt sich aus dem Produkt I x t die Lichtmenge M.

In Fig.1b ist das Schaubild der Strahlung einer gepulsten Lichtquelle dargestellt, wobei über das gleiche Zeitintervall t vier Hellzeiten P₁-P₄ mit der gleichen Intensität I von drei Dunkelzeiten D₁-D₃ abgewechselt werden. Dadurch würde sich mit den dargestellten Größen eine Summe der Teillichtmengen M₁+M₂+M₃+M₄ ergeben, welche 3/7 kleiner als die Lichtmenge M ist.

(Gestützt auf die Erkenntnisse von Talbot-Plateau führt diese Reduktion jedoch nicht zu einer Reduktion des Sehreizes (im Betrachterauge), sofern die Frequenz über der Flimmergrenze liegt. Daher sieht der Beobachter trotz geringerer Lichtmenge gemäß Fig.1b gleich viel wie bei einer Beleuchtung nach Fig.1a.)
ln der dargestellten Form sind die Hellzeiten Pₓ mit den Dunkelzeiten Dₓ nicht symmetrisch, d.h. die Hellzeiten dauern in der dargestellten Form länger als die Dunkelzeiten. Bei gleich lang dauernden, also gleich breiten Intervallen auf der Zeitachse t, wären sie symmetrisch darzustellen. Wie schon erwähnt, ist dieses jedoch für die Wahrnehmung eines vermeintlich kontinuierlichen Lichts mit der Lichtmenge M = M₁+M₂+M₃+M₄+Mₙ unerheblich, solange das "Aufflackern" der Hellzeiten über der physiologischen Flimmergrenze liegt.

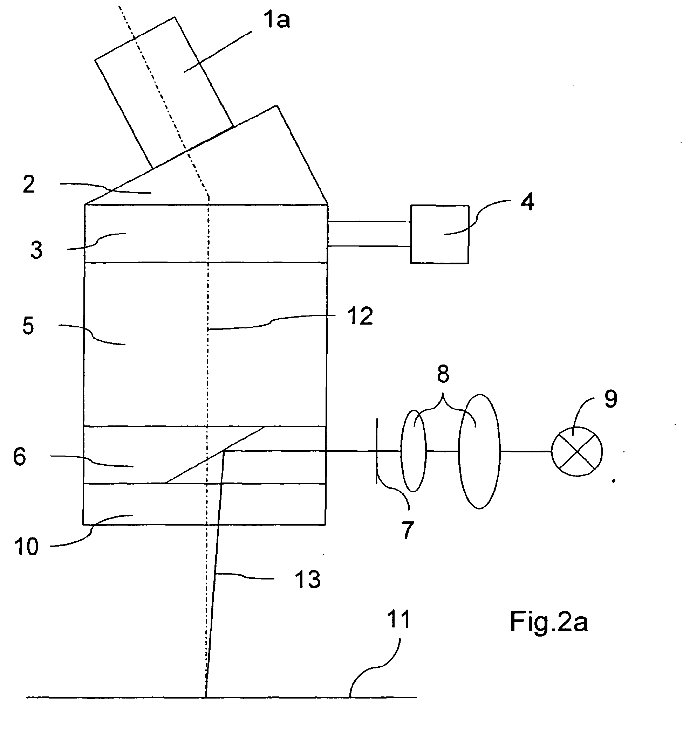

In Fig.2a ist der schematische Aufbau eines Operationsmikroskops dargestellt. Durch einen Binokulartubus 2 mit Okularen 1a, b (Okular 1b verdeckt), durch einen optischen Teiler 3 (in welchen eine optionale Kamera 4 integriert ist), durch ein (optionales) Zoom 5, durch eine Beleuchtungseinheit 6 und durch ein Hauptobjektiv 10 wird ein Beobachtungsstrahlengang mit der Achse 12 geführt. Bei einer stereoskopischen Ausführung des Mikroskops liegen rechter und linker Beobachterstrahlengang in der schematischen Seitenansicht aller Zeichnungen hintereinander. Durch das Hauptobjektiv 10 zu diesem Beobachtungsstrahlengang wird mittels der Beleuchtungseinheit 6 ein gepulster Beleuchtungsstrahl 13 eingespiegelt. Die Lampe 9 sendet das Beleuchtungslicht aus, das durch die Beleuchtungsoptik 8 ins Objektfeld 11 abgebildet wird, in welchem das z.B. zu beobachtende Patientenauge liegt. Die Pulsation des Lichtstrahls 13 wird im vorliegenden Fall durch einen Shutter 7 erzeugt. Im Falle der Verwendung einer Stroboskoplampe 9a anstelle einer kontinuierlich strahlenden Lampe 9 ist die Anordnung des Shutterrades im Prinzip überflüssig, soll jedoch zur Optimierung der Beleuchtung des Stroboskops nicht ausgeschlossen sein.

Der Vorteil einer herkömmlichen Beleuchtung, welche mittels eines Shutterrades getaktet ist, liegt in der guten Lichtqualität gekennzeichnet durch einen der visuellen Beobachtung angepassten Spektralbereich. Dabei wird hier ausdrücklich nicht ausgeschlossen, dass auch Lampen, getaktet oder kontinuierlich, mit nicht sichtbaren Spektralbereichen verwendet werden.

Die in allen Zeichnungen dargestellte Durchdringung von Beobachter- und Beleuchtungsstrahlengang ist nicht zwingend erforderlich. Denkbare Beispiele stellen auch eine partielle Durchdringung oder sogar eine strikte Trennung beider Strahlengänge dar.

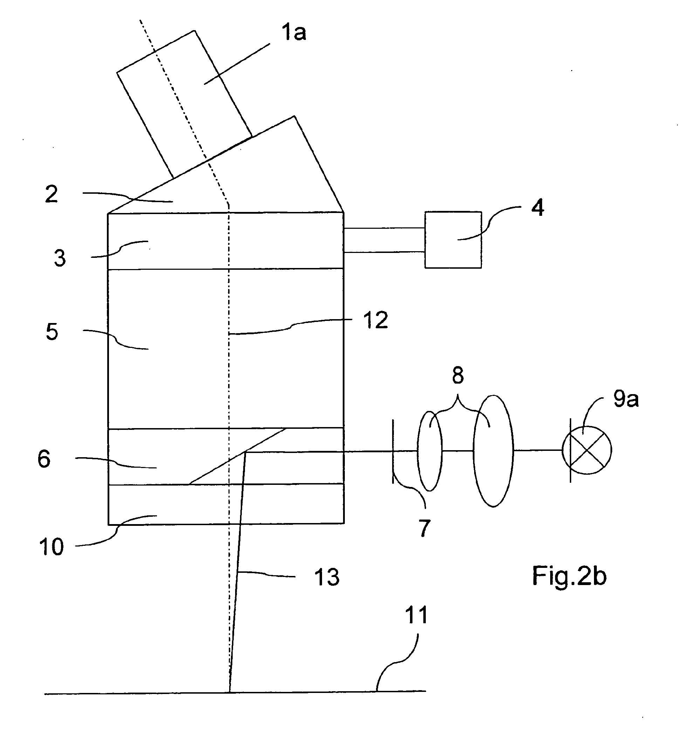

In Fig.2b ist der gleiche Aufbau eines Mikroskops wie in Fig.2a dargestellt, nur mit dem Unterschied, dass statt der herkömmlichen Beleuchtung 9 eine Stroboskoplampe 9a die gepulste Beleuchtung erzeugt. Die Anordnung des Shutters 7 ist optional.

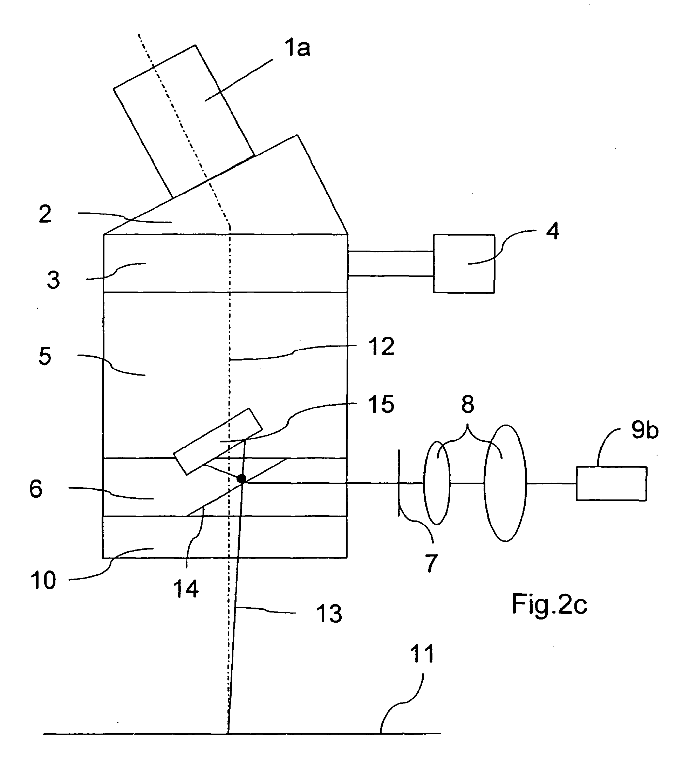

In Fig.2c ist schematisch ein Ausführungsbeispiel dargestellt, bei dem in diesem Fall ein Laser 9b, z.B. ein Weißlichtlaser, die Funktion der Beleuchtung übernimmt und seinen gebündelten Lichtstrahl auf einen optomechanischen Spiegel 14 wirft. Der optomechanische Spiegel 14 wird mittels dem Motor 15 so präzise bewegt, dass der reflektierte Lichtstrahl 13 eine Scanbewegung ausführt. Wenn dabei die Scanzyklen so rasch aufeinander folgen, das daraus eine Frequenz über der Flimmergrenze resultiert, so nimmt ein Beobachter durch den Binokulartubus 2 eine unverminderte Ausleuchtung des Objektfeldes 11 wahr. Die Anordnung der Beleuchtungsoptik 8 und des Shutters 7 sind optional.

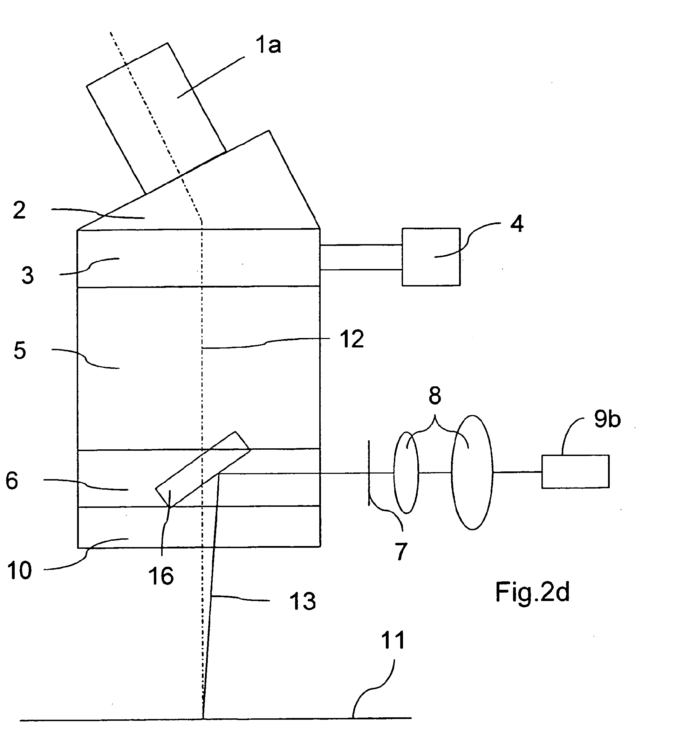

Fig.2d zeigt einen Aufbau mit einem Laser 9b und einem symbolisch dargestellten Mikrospiegel 16, welcher die Scanbewegung des Lichtstrahls 13 erzeugt. Auch hier sind die Anordnung der Beleuchtungsoptik 8 und des Shutters 7 optional.

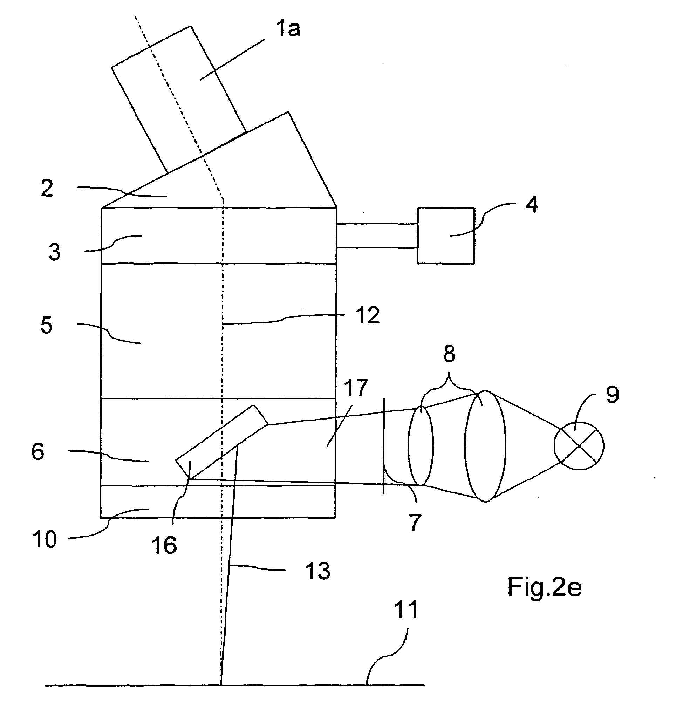

In Fig.2e ist ein Aufbau mit einer herkömmlichen Beleuchtung 9, mit Beleuchtungsoptik 8 und einem optionalen Shutter 7 dargestellt. Hierbei ist gezeigt, dass es für den Mikrospiegel 16 keines gebündelten Lichtstrahls bedarf, sondern dass eine flächige Beleuchtung mit einem Lichtbüschel 17 erfolgt und das Scannen des Lichtstrahls 13 im Objektfeld 11 durch das sukzessive Aktivieren der einzelnen Spiegelelemente des Mikrospiegels 16 erfolgt.

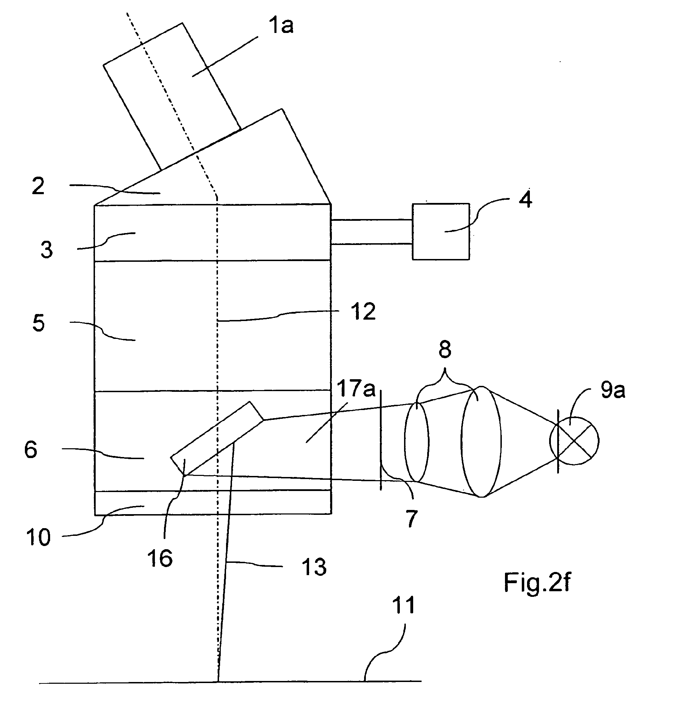

Fig.2f zeigt, dass das Lichtbüschel 17, sei es aufgrund der Pulserzeugung durch die Stroboskoplampe 9a oder durch den Shutter 7 oder beides, den Mikrospiegel auch gepulst treffen kann, sodass ein gepulster Lichtstrahl die Scanbewegung vollführt.

### Bezugszeichenliste

- 1a,b -: Okular
- 2 -: Binokulartubus
- 3 -: Optischer Teiler
- 4 -: Kamera
- 5 -: Zoom
- 6 -: Beleuchtungseinheit
- 7 -: Shutter
- 7a -: Shutterrad
- 7b -: Elektrochromer Shutter
- 8 -: Beleuchtungsoptik
- 9 -: Lampe
- 9a -: Stroboskoplampe
- 9b -: Laser
- 10 -: Hauptobjektiv
- 11 -: Objektfeld
- 12 -: Achse des Beobachtungsstrahlengangs
- 13 -: Beleuchtungsstrahl
- 14 -: Optomechanischer Spiegel
- 15 -: Motor
- 16 -: Mikrospiegel
- 17 -: Lichtbüschel
- 17a -: Gepulstes Lichtbüschel

- I -: Intensität
- t -: Zeitintervall
- M -: Lichtmenge
- M_{1,2,3,4} -: Teillichtmenge
- P_{1,2,3,4} -: Hellzeit
- D_{1, 2,3}-: Dunkelzeit

## Patentansprüche

1. Ophthalmo-Operationsmikroskop mit Okularen (1a, 1b), einem Binokulartubus (2), einem optischen Teller (3) für eine Kamera (4), einem Zoom (5), einem Hauptobjektiv (10), einem Beobachtungsstrahlengang (12) und einer zwischen dem Hauptobjektiv (10) und dem Zoom (5) angeordneten Beleuchtungseinheit (6), mittels derer in den Beobachtungs-Strahlengang (12) ein Beleuchtungsstrahl (13) von einer Lichtquelle (9) einspiegelbar ist, **dadurch gekennzeichnet, dass** die Lichtquelle (9) zur Abgabe eines gebündelten Beleuchtungsstrahls (13) ausgebildet ist, der mittels eines in der Seleuchtungseinheit (6) angeordneten und durch einen Motor (15) betriebenen optomechanischen Spiegels (14) periodisch wiederholt oberhalb der Flimmerverschmelzungsgrenze Ober ein Objektfeld (11) scanbar ist.

2. Ophthalmo-Operationsmikroskop mit Okularen (1a, 1b), einem Binokulartubus (2), einem optischen Teller (3) für eine Kamera (4), einem Zoom (5), einem Hauptobjektiv (10), einem Beobachtungsstrahlengang (12) und einer zwischen dem Hauptobjektiv (10) und dem Zoom (5) angeordneten Beleuchtungseinheit (6), mittels derer in den Beobachtungs-Stmhlengang (12) ein Beleuchtungsstrahl (13) von einer Lichtquelle (9) einspiegelbar ist, **dadurch gekennzeichnet, dass** die Lichtquelle (9) zur Abgabe des Beleuchtungsstrahls (13) ausgebildet ist, der durch ein in der Beleuchtungseinheit (6) angeordnetes Mikrospiegel-Arr-ay (16) periodisch wiederholt scanbar ist, wobei das Mikrospiegel-Array (16) im Betriebszustand durch reihenweise Betätigung einzelner Mikrospiegel die Scan-Bewegung des Seleuchtungsblrahls (13) erzeugt und wobei die periodische Wiederholung oberhalb der Flimmerverschmelzungsgrenze liegt.

3. Ophthalmo-Operationsmlkroskop nach Anspruch 1 oder 2, **dadurch gekennzeichnet, dass** die Lichtquelle (9) zur Abgabe gepulsten Lichts mit einer Frequenz oberhalb der Flimmerverschmelzungsgrenze ausgebildet ist.

4. Ophthalmo-Operationsmikroskop nach Anspruch 1, **dadurch gekennzeichnet, dass** die Lichtquelle (9) ein Laser (9b) ist.

5. Ophthalmo-Operationsmikroskop nach Anspruch 3, **dadurch gekennzeichnet, dass** die Lichtquelle (9) eine Stroboskoplampe (9a) ist.

6. Ophthalmo-Operationsmikroskop nach einem der vorhergehenden Ansprüche, **dadurch gekennzeichnet, dass** eine Messvorrichtung für das Messen der Anzahl der Lichtimpulse und/oder deren Intensität vorgesehen ist, und diese Messwerte für den Beobachter anzeig- und dokumentierbar sind.

7. Ophthalmo-Operationsmikroskop nach einem der vorhergehenden Ansprüche, **dadurch gekennzeichnet, dass** es einen afokalen Strahlengang umfasst.

8. Ophthalmo-Operationsmikroskop nach einem der vorhergehenden Ansprüche, **dadurch gekennzeichnet, dass** es einen Strahlengang nach Greenough-System umfasst.

9. Ophthalmo-Operationsmlkroskop nach Anspruch 3, **dadurch gekennzeichnet, dass** Ober einen Strahlenteller eine CCD-Kamera an den Beleuchtungsstrahlengang (13) angeschlossen ist, und dass die Auslesefrequenz der CCD-Kamera mit dem gepulsten Beleuchtungsstrahl synchronisiert ist.

10. Ophthalmo-Operationsmikroskop nach Anspruch 3, **dadurch gekennzeichnet, dass** die Einspiegelung von Bildinformationen oder externer Bilderzeuger mit der PulsFrequenz des Beleuchtungsstrahls (13) synchronisiert ist.

## Claims

1. Ophthalmic operation microscope having eyepieces (1a, 1b), a binocular tube (2), an optical splitter (3) for a camera (4), a zoom (5), a main objective (10), an observation beam path (12) and an illumination unit (6) arranged between the main objective (10) and the zoom (5), by means of which an illumination beam (13) from a light source (9) can be reflected into the observation beam path (12), **characterized in that** the light source (9) is designed to output a collimated illumination beam (13) which can be scanned periodically repeatedly above the flicker fusion threshold over an object field (11) by means of an optional-mechanical mirror (14) arranged in the illumination unit (6) and driven by a motor (15).

2. Ophthalmic operation microscope having eyepieces (1a, 1b), a binocular tube (2), an optical splitter (3) for a camera (4), a zoom (5), a main objective (10), an observation beam path (12) and an illumination unit (6) arranged between the main objective (10) and the zoom (5), by means of which an illumination beam (13) from a light source (9) can be reflected into the observation beam path (12), **characterized in that** the light source (9) is designed to output the illumination beam (13) which can be scanned periodically repeatedly via a micromirror array (16) arranged in the illumination unit (6), the micromirror array (16) generating the scan movement of the illumination beam (13) by line-wise actuation of individual micromirrors in the operating state and the periodic repetition lying above the flicker fusion threshold.

3. Ophthalmic operation microscope according to Claim 1 or 2, **characterized in that** the light source (9) is designed to output pulsed laser light with a frequency above the flicker fusion threshold.

4. Ophthalmic operation microscope according to Claim 1, **characterized in that** the light source (9) is a laser (9b).

5. Ophthalmic operation microscope according to Claim 3, **characterized in that** the light source (9) is a stroboscopic lamp (9a).

6. Ophthalmic operation microscope according to one of the preceding claims, **characterized in that** a measuring device is provided for measuring the number of light pulses and/or their intensity, and these measurement values can be displayed for the user and documented.

7. Ophthalmic operation microscope according to one of the preceding claims, **characterized in that** it comprises an afocal beam path.

8. Ophthalmic operation microscope according to one of the preceding claims, **characterized in that** it comprises a beam path according to the Greenough system.

9. Ophthalmic operation microscope according to Claim 3, **characterized in that** a CCD camera is connected to the illumination beam path (13) via a beam splitter, and **in that** the readout frequency of the CCD camera is synchronized with the pulsed illumination beam.

10. Ophthalmic operation microscope according to Claim 3, **characterized in that** the overlay of image information or external image generators is synchronized with the pulse frequency of the illumination beam (13).

## Revendications

1. Microscope opératoire ophtalmologique, comprenant des oculaires (1a, 1b), un tube binoculaire (2), un séparateur optique (3) pour une caméra (4), un objectif à focale variable (5), un objectif principal (10), une trajectoire des faisceaux d'observation (12) et une unité d'éclairage (6) disposée entre l'objectif principal (10) et l'objectif à focale variable (5), au moyen de laquelle un faisceau d'éclairage (13) provenant d'une source de lumière (9) peut être projeté dans la trajectoire des faisceaux d'observation (12), **caractérisé en ce que** la source de lumière (9) est réalisée pour délivrer un faisceau d'éclairage focalisé (13) qui peut être amené à balayer périodiquement à plusieurs reprises un champ objet (11) au-dessus du seuil de fusion du papillotement au moyen d'un miroir optomécanique (14) disposé dans l'unité d'éclairage (6) et commandé par un moteur (15).

2. Microscope opératoire ophtalmologique, comprenant des oculaires (1a, 1b), un tube binoculaire (2), un séparateur optique (3) pour une caméra (4), un objectif à focale variable (5), un objectif principal (10), une trajectoire des faisceaux d'observation (12) et une unité d'éclairage (6) disposée entre l'objectif principal (10) et l'objectif à focale variable (5), au moyen de laquelle un faisceau d'éclairage (13) provenant d'une source de lumière (9) peut être projeté dans la trajectoire des faisceaux d'observation (12), **caractérisé en ce que** la source de lumière (9) est réalisée pour délivrer un faisceau d'éclairage (13) qui peut être amené par un réseau de micromiroirs (16) disposé dans l'unité d'éclairage (6) à effectuer un balayage périodiquement à plusieurs reprises, dans lequel le réseau de micromiroirs (16) génère à l'état de fonctionnement le mouvement de balayage du faisceau d'éclairage (13) par un actionnement en série de micromiroirs individuels, et dans lequel la répétition périodique se situe au-dessus du seuil de fusion du papillotement.

3. Microscope opératoire ophtalmologique selon la revendication 1 ou 2, **caractérisé en ce que** la source de lumière (9) pour délivrer de la lumière pulsée est réalisée à une fréquence au-dessus du seuil de fusion du papillotement.

4. Microscope opératoire ophtalmologique selon la revendication 1, **caractérisé en ce que** la source de lumière (9) est un laser (9b).

5. Microscope opératoire ophtalmologique selon la revendication 3, **caractérisé en ce que** la source de lumière (9) est une lampe stroboscopique (9a).

6. Microscope opératoire ophtalmologique selon l'une quelconque des revendications précédentes, **caractérisé en ce qu'**un dispositif de mesure destiné à mesurer le nombre des impulsions lumineuses et/ou leur intensité est prévu et que ces valeurs mesurées peuvent être affichées et documentées pour l'observateur.

7. Microscope opératoire ophtalmologique selon l'une quelconque des revendications précédentes, **caractérisé en ce qu'**il comprend une trajectoire des faisceaux afocale.

8. Microscope opératoire ophtalmologique selon l'une quelconque des revendications précédentes, **caractérisé en ce qu'**il comprend une trajectoire des faisceaux selon le système de Greenough.

9. Microscope opératoire ophtalmologique selon la revendication 3, **caractérisé en ce qu'**une caméra CCD est connectée à la trajectoire des faisceaux d'observation (13) par l'intermédiaire d'un séparateur de faisceaux, et que la fréquence de lecture de la caméra CCD est synchronisée sur le faisceau d'éclairage pulsé.

10. Microscope opératoire ophtalmologique selon la revendication 3, **caractérisé en ce que** la projection d'informations d'image ou de générateurs d'image externes est synchronisée sur la fréquence d'impulsion du faisceau d'éclairage (13).
